# EUROPEAN PATENT SPECIFICATION

(11) **EP 3 297 683 B1**
(45) Date of publication and mention of the grant of the patent: **23.03.2022**
(21) Application number: 16723477.2
(22) Date of filing: 13.05.2016
(51) Int. Cl.: A61K 49/00, A61K 47/10, G01N 33/15, A61K 31/167, C07C 231/02, C07C 231/12, A61P 9/10

(54) **IDENTIFIABLE CHEMICAL PRODUCT**
INDENTIFIZIERBARES CHEMISCHES PRODUKT
PRODUIT CHIMIQUE IDENTIFIABLE

(30) Priority: 20.05.2015 GB 201508668
(43) Date of publication of application: 28.03.2018
(73) Proprietor: Johnson Matthey Public Limited Company, London EC4A 4AB (GB)
(72) Inventor: CROUD, Vincent Brian, Billingham, Cleveland TS23 4ED (GB); MALTAS, Philip James, Billingham, Cleveland TS23 4ED (GB); MCCALLIEN, Duncan William John, Billingham, Cleveland TS23 4ED (GB)
(74) Representative: Atkinson, Ian Anthony
(86) International application number: PCT/GB2016/051379
(87) International publication number: WO 2016/185177

(56) References cited:
- WO-A1-2014/058582
- US-A1- 2006 054 506
- US-A1- 2008 305 489
- M.-C. FOURNIER ET AL: "A new parallel competing reaction system for assessing micromixing efficiency-Experimental approach", CHEMICAL ENGINEERING SCIENCE, vol. 51, no. 22, 1 November 1996 (1996-11-01), pages 5053-5064, XP055293246, GB ISSN: 0009-2509, DOI: 10.1016/0009-2509(96)00270-9

## Description

The present invention concerns a process for manufacturing a chemical composition which may be identified and distinguished from similar chemical products.

It is known that a composition or product may be identified by adding to the composition a marker compound. Identification of the marker compound may then be used to identify a particular composition which has originated from the source at which the marker compound was added. Commonly used markers include dyes. A problem with using the conventional method of adding a marker to a composition is that the marker may separate from the composition during subsequent processing of the composition. It is an object of the invention to provide a solution to this problem.

The present invention relates to a method for marking a composition comprising a chemical product C. The process of the present invention is defined in claim 1.

Also disclosed is a composition comprising an intimate mixture of:
(a) a chemical product C and
(b) a marker product AM;
wherein marker product AM is formed by reaction of a chemical intermediate A with a marker chemical M and chemical compound C is formed by reaction of chemical intermediate A with a chemical intermediate B. Chemical product C may be formed in the same reaction mixture as marker product AM. The composition may further comprise a marker product CM which is formed by reaction of a chemical product C with a marker chemical M. The composition may consist essentially of chemical product C, marker product AM, optionally marker product CM and further optionally an additional marker as hereinafter described. The composition as such is not part of the present invention.

Chemical product C is a chemical product synthesised from intermediate A and intermediate B which is desired to be marked with marker product AM and/or marker product CM so that it is subsequently identifiable. Chemical product C may, following manufacture, be mixed with other compounds to form a composition. The composition may be a functional composition, such as a biologically active composition, for example a biocide or a medicament, a food or beverage, or a perfume. Chemical product C may be an active or a non-active compound in such a composition. Thus chemical product C may itself have activity as an active compound such as a pesticide, a herbicide, a medicament, a food additive, an aroma compound, a dye, a pigment, a plant product or a fuel additive, for example. Chemical product C may be a non-active ingredient in such a composition, such as a diluent, excipient or filler. Chemical product C may be an intermediate which may be used to form an active or non-active compound by further reaction. Chemical product C may comprise a liquid or solid bulk material such as a solvent, a fuel, a polymer or wood derivative. Chemical product C may have a functional group capable of reacting with marker chemical M to form marker product CM under the conditions present in the reaction mixture.

Chemical intermediate B is a chemical compound which is capable of reacting with intermediate A to form a chemical product C. Chemical intermediate B incorporates a functional group capable of reacting with chemical intermediate A to form chemical product C. Marker chemical M may incorporate the same type of functional group as that in intermediate B or the functional group in marker chemical M which is capable of reacting with intermediate A or chemical product C may be different from that in intermediate B. The functional groups on intermediate B and marker M which are capable of reacting with intermediate A, or on marker M which is capable of reacting with chemical product C, may be selected from any of a wide variety of chemical functional groups. The functional group present in intermediate B and/or marker chemical M which is capable of reacting with a functional group present in intermediate A, or present in marker chemical M which is capable of reacting with a functional group present in chemical product C, may be selected from an acid halide, an alcohol, an aldehyde, an amide, an amine, a carboxylate, a carboxylic acid, a cyanate, an ether, an ester, a Grignard, an isocyanate, a ketone, a nitrile, a nitro, an acidic phosphorus-containing group, such as a phosphonic acid, a phosphine, a sulfide, a sulfone, a sulfonic acid, a sulfoxide, a thiol, a cross-coupling species (such as aryl/alkenyl/alkyl-halides, aryl/alkenyl/alkyl-boronic acids, stannanes or Grignard species); or any other suitable chemical functional group. The functional group present in intermediate A which is capable of reacting with a functional group present in intermediate B and/or marker chemical M may be selected from the same groups as those listed for intermediate B and/or marker chemical M. The functional group in intermediate B for reaction with intermediate A may be the same as or may be different from the functional group in marker chemical M which is intended to react with intermediate A.

Selection of any particular functional group is guided by knowledge of the type of functional group which can react with intermediate A in the desired manner. The skilled chemist who is knowledgeable about chemical product C and its manufacture would already have selected intermediate B which is most appropriate for the manufacture of chemical product C. The selection of marker chemical M may therefore be made based on knowledge of the type of functional group required to react with intermediate A. The selection of marker chemical M should ensure that the resulting marker AM may have appropriate properties considering the intended use of product C. Therefore when product C is intended to be non-toxic, marker product AM should also be non-toxic at the concentration at which it is intended to be present in a final product.

Marker chemical M may be a chemical compound which is capable of reacting with intermediate A to form a marker product AM. Marker chemical M may alternatively be a chemical compound which is capable of reacting with product C to form a marker product CM. Marker chemical M may be capable of reacting with intermediate A to form a marker product AM and also with chemical product C to form marker product CM. Marker product AM and marker product CM are chemical compounds capable of forming an intimate well-dispersed physical mixture with chemical product C and which are identifiable by analysis within said mixture. Marker chemical M may be selected to be capable of reacting with intermediate B in addition to intermediate A or product C. In such a case a marker product BM may be formed in addition to marker product AM and/or marker product CM. If a marker product BM is formed then it may be identified by means of any of the detection methods described herein.

Marker chemical M is selected so that marker product AM or CM may be distinguished and identified in a mixture containing chemical product C and AM or CM and any other compounds which may be present in a composition containing chemical product C and AM/CM when such mixture or composition is analysed. AM and CM are therefore detectable by an analytical method. AM and CM are preferably detectable at a concentration in the mixture or in a final product containing the mixture in a diluted form at less than 1% by weight. AM and CM are more preferably detectable at a concentration in the mixture less than 0.01% by weight. Most preferably the marker AM and/or CM is detectable in the mixture at a concentration as low as 1 ppb by weight. The analytical method may be selected from any suitable method. Preferred methods include spectroscopic methods and chromatographic methods. Such methods include but are not limited to spectrophotometric absorption in the ultraviolet, visible, near infrared or mid-infrared regions; fluorescent emission spectroscopy; nuclear magnetic resonance spectroscopy, electron spin resonance spectroscopy, Raman spectroscopy, resonance Raman spectroscopy, surface-enhanced Raman spectroscopy (SERS); surface-enhanced resonance Raman spectroscopy (SERRS), tip enhanced Raman spectroscopy, spatially off-set Raman spectroscopy. Suitable chromatographic methods include gas chromatography and liquid chromatography (LC), including HPLC, in each case, coupled with a suitable detection method such as mass spectroscopy, infra-red spectroscopy, electron capture detection, UV spectroscopy, evaporative light scattering detection (ELSD) or any other suitable detection technique. If the marker AM and/or CM and product C can be distinguished and determined quantitatively, then the relative or absolute amount of AM and/or CM and product C may be used as an additional identification for a product containing product C. Quantitative detection of marker AM and/or CM and product C may allow dilution of a product to be detected. The marker AM or CM may be separated from a sample of the mixture with product C before or during analysis. A sample of the mixture of marker AM /CM with product C may be subjected to concentration or derivatisation techniques before or during analysis.

Chemical intermediate A is a chemical compound which is capable of reacting with intermediate B to form a chemical product C. Chemical intermediate A may, in one embodiment, also be capable of reacting with marker chemical M to form a product AM. In this embodiment, intermediate A must be capable of reacting with intermediate B and also with marker M so that when intermediate A is reacted with a mixture of intermediate B and marker M, a product mixture comprising product C and a marker compound AM is formed. In another embodiment, chemical product C is capable of reacting with marker chemical M to form a product CM. The relative proportion of product C to AM or CM formed in the reaction mixture may be determined by the amount of marker M in the reaction mixture, although this is not necessarily the case if each of marker M and intermediate B reacts with intermediate A at a different rate or if product C and AM have different chemical properties which influence their stability. A mixture of Product C with AM or CM may be analysed following manufacture of product C by the method of the invention to determine the concentration of marker AM or marker CM in the original product mixture. If a product suspected of containing product C is analysed at a subsequent step in the supply chain of the product C, a different ratio of the marker products AM or CM to product C from that measured immediately after manufacture may indicate replacement or dilution of product C following its manufacture.

The mixture of chemical product C and the marker products AM or CM separated from the reaction mixture in step (ii) is preferably a homogeneous mixture. Marker products AM or CM should be distributed evenly throughout the mixture with product C. Marker products AM and CM may have similar physical properties to product C. That is, the density, colour, particle size, crystallinity and/or any other relevant physical property of the marker product may be similar to the respective property of product C. If the physical properties are similar then the separation of the marker products from the mixture with product C during normal commercial handling and use of product C may be minimised. This helps to ensure that when the mixture is processed through subsequent steps to form a distributed final product, the mixture of product C with the marker product AM or CM is retained in its original composition. If, for example, marker product AM is significantly heavier or has a different density from product C, it may become partially separated during subsequent processing. Handling of the product C with marker AM/CM under conditions such that the differences in any physical properties between the species is maximised should be avoided. Separation of the mixture of product C and marker product(s) AM /CM during handling and commercial use of product C should be avoided.

Marker product AM or CM preferably has similar chemical properties to product C. It is not necessary for a marker product to have the same biological or chemical activity or effect as product C. However, the solubility, partition coefficient, melting point, boiling point, reaction to alkali or reaction to acid may be similar to those of product C to prevent, so far as possible, any separation of the mixture during subsequent process steps in which product C is handled before distribution as a finished product.

The mixture may contain from 1 ppb by weight to 1% by weight of marker product AM or CM based on the total weight of chemical product C and marker product AM/CM. The marker chemical M may be used in an amount which is selected to provide a unique identifier for one of a plurality of mixtures of chemical product C with marker product AM or CM. In that way, for example, an individual batch of product C may be distinguished from another batch of product C by analysing the material to determine the amount of marker product present. Measurement of the absolute amount of a marker product in a batch, or of the relative amounts of the marker products and product C, may be used to identify the batch of product C from which a sample has been drawn.

More than one marker may be present in the mixture. At least two marker chemicals M1, M2 ...Mn may be present in the reaction mixture, each marker chemical forming a different marker product by reaction with chemical intermediate A or chemical product C and each said different marker product being distinguishable from chemical product C and, preferably, also from each other marker product, by analysis. n represents the number of marker chemicals M present in the mixture, n may be up to 100, for example 2 - 10 or 2 - 20. For example, if two marker chemicals, M1 and M2 are present in the reaction mixture and each is capable of reacting with chemical intermediate A, then two marker products AM1 and AM2 (or CM1, CM2) will be formed in addition to chemical product C. The relative proportions of marker chemicals M1 - Mn may be calculated to provide an identifiable signal ratio of each resulting marker product AM when the mixture is analysed. The signal ratio of any marker product to any other different marker product in the mixture may provide an identifiable signal to identify originally manufactured product C. The signal ratio of any marker product to product C in the mixture may provide an identifiable signal to identify originally manufactured product C. When more than one marker chemical M is used in the reaction mixture, the marker chemicals may be used in relative proportions which are selected to provide a unique signal ratio for one of a plurality of mixtures of chemical product C and marker product AM or CM. In that way an individual batch of product C may be distinguished from another batch of product C by analysing the material for the marker products present in each batch. When n different marker chemicals M have been selected to provide different marker products in different predetermined proportions in each of at least two batches of product C, measurement of the relative amounts of the marker products may be used to identify the batch of product C from which a sample has been drawn.

The mixture comprising chemical product C may comprise one or more additional markers or tags which are different from CM or AM. The additional markers may comprise detectable chemicals such as visible dyes, fluorescent dyes, chemicals detectable by chemical analysis, spectroscopy or chromatography associated with a suitable detector (e.g. mass spectroscopy) etc. Suitable additional markers may be selected from the type of markers or tags known for marking such chemical compositions. The additional marker(s) may be added to a mixture containing chemical product C and marker product AM and/or CM. The additional marker(s) may be intimately mixed with a mixture containing chemical product C and marker product AM and/or CM. An additional marker may be located on a container, for example packaging, containing a mixture comprising chemical product C.

To summarise the nomenclature used in this patent application:
(chemical) intermediate A = a chemical compound which is capable of reacting with intermediate B to form a chemical product C, and which may also be capable of reacting with marker chemical M to form a product AM.
(chemical) intermediate B = a chemical compound which is capable of reacting with intermediate A to form a chemical product C.
Chemical product C = a chemical product synthesised from intermediate A and intermediate B which is desired to be marked with a marker product so that it is subsequently identifiable. Marker chemical M = a chemical compound which is capable of reacting with intermediate A to form a marker product AM or with chemical product C to form marker product CM or which is capable of reacting with both intermediate A and chemical product C.

Marker product AM = a chemical compound formed by the reaction of marker chemical M with intermediate A and which is capable of forming an intimate mixture with chemical product C and which is identifiable within said mixture by analysis.

Marker product CM = a chemical compound formed by the reaction of marker chemical M with chemical product C and which is capable of forming an intimate mixture with chemical product C and which is identifiable by analysis within said mixture.

Marker product: either marker product AM or marker product CM or a mixture of marker products AM and CM;

Marker product AM/CM: either marker product AM or marker product CM or a mixture of marker products AM and CM;

As an example of how the invention may be embodied, chemical product C may be an amide such as DEET (N,N-Diethyl-3-methyl-benzamide). Amide compounds may be synthesised from the reaction of an amine with an acyl chloride. If it is desired to mark DEET by the method of the invention, chemical intermediate A may be m-toluoyl chloride and chemical intermediate B may be diethylamine. Marker chemical M may be selected from an amine having a functional group which is readily detectable and distinguishable at low concentration by a simple analytical method such as surface enhanced Raman spectroscopy (SERS) so that after reaction with intermediate A to produce AM the marker product AM is detectable and distinguishable from product C by SERS. An example of a suitable amine compound is 3-ethynyl-phenylamine. If, during the synthesis of DEET (N,N-Diethyl-3-methyl-benzamide), m-toluoyl chloride (chemical intermediate A) is reacted with a mixture of 99 mol% diethylamine (chemical intermediate B) and 1 mol% 3-Ethynyl-phenylamine (marker M), then the product contains 99 mol% DEET (N,N-Diethyl-3-methyl-benzamide) (chemical product C) and 1 mol% N-(3-Ethynyl-phenyl)-3-methyl-benzamide (marker product AM). Such a mixture is then identifiable by analysis using SERS, because the ethynylphenyl functionality of the marker AM is detectable by SERS, yielding a spectrum which is distinguishable from the spectrum of DEET.

The invention will be further described in the following examples.

### Example 1: Synthesis of marked DEET

Diethylamine (0.248 g, 3.40 mmol) and 3-ethynyl-phenylamine (0.4 mg, 0.0032 mmol) (from stock solution in CHCl₃) were added to a solution of m-toluoyl chloride (0.50 g, 3.23 mmol) in CHCl₃ (5 ml). Triethylamine (TEA) (0.86 ml, 4.85 mmol) was added and the mixture stirred at room temperature for 2 hours. The reaction was quenched with water (5 ml), diluted with dichloromethane (10 ml) and the organic phase was separated and washed with HCI (15%, 10 ml) then brine (5 ml). The organic phase was dried (MgSO₄) and concentrated in vacuo to afford DEET (0.58 g, 3.03 mmol, 94 %) tagged at 0.1 mol % as a colourless oil.

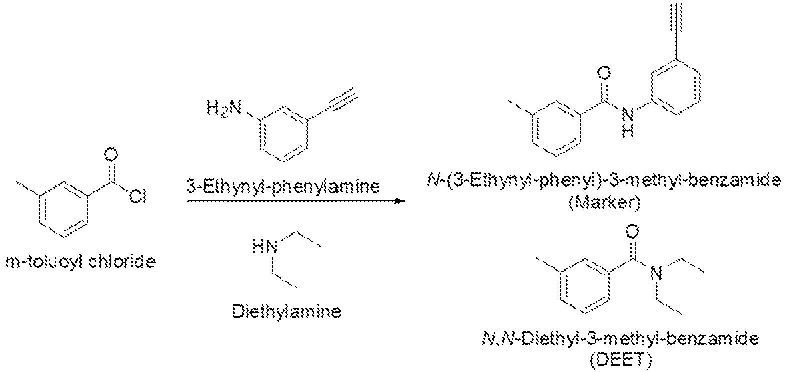

An untagged DEET compound was made by the same method but omitting the 3-ethynyl-phenylamine from the reaction mixture. The product was confirmed by GC-Mass spectroscopy and NMR spectroscopy.

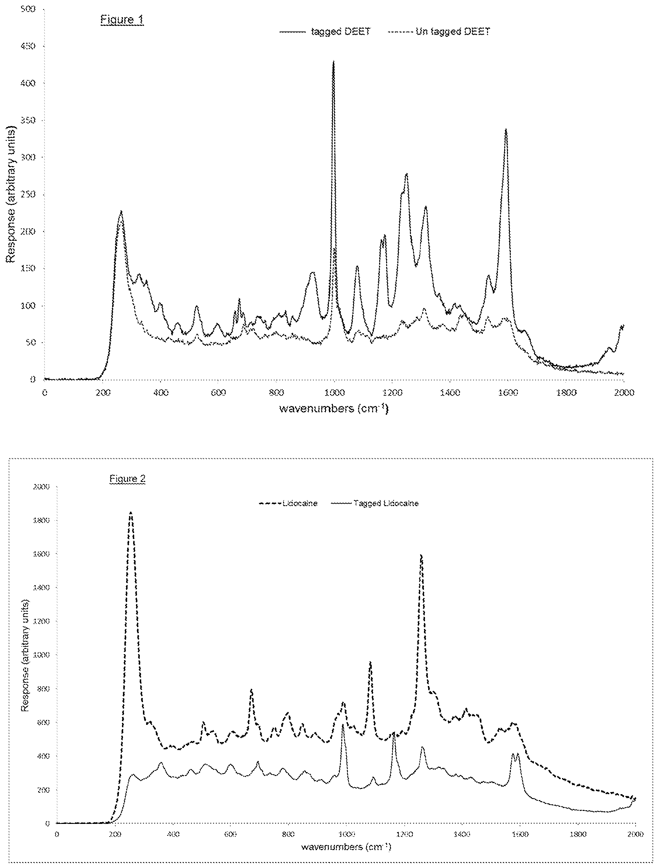

The SERS response was detected by the following method. 10 mg of either untagged DEET or the tagged DEET mixture prepared in Example 1 was taken up in water (2 ml) and shaken to form a stock solution. Gold colloid (500 µL, 60.7 nm in citrate buffer, 0.01% w/w supplied by Cabot) and deionised water (300 µL) were added to 100 µL of the above stock solution and the mixture shaken. NaCl (100 µL, 5% aq.) was added, the mixture shaken and the SERS response of the resulting solution was then collected using an Ocean Optics 785 nm laser (QE65000, 1000 ms integration, 5 iterations). The SERS spectra are shown in Fig 1. Untagged DEET shows a weak SERS band at 995 cm-1. Distinct SERS bands can be seen in the tagged samples at 1161, 1252, 1303 and 1595 cm-1 (in addition to a signal at 995 cm-1). The tagged DEET product mixture is therefore readily detectable using SERS and may be identified as a compound made using the method of the invention.

### Example 2: Synthesis of marked Lidocaine

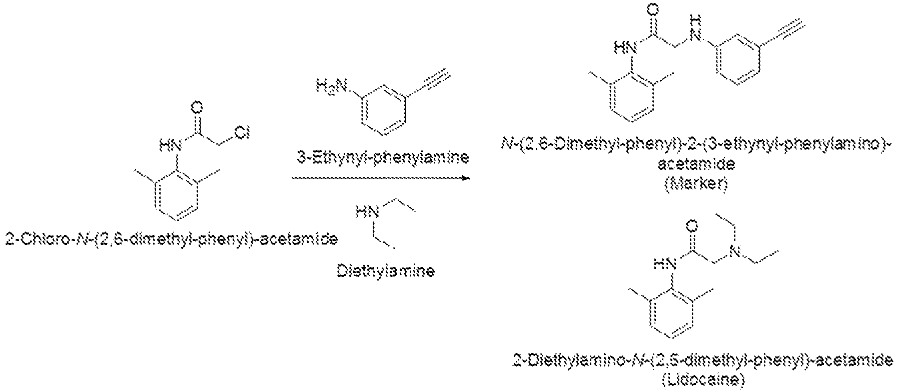

Potassium iodide (0.18 g, 1.07 mmol) was added to a solution of 2-chloro-N-(2,6-dimethylphenyl)-acetamide (0.212 g, 1.07 mmol), diethylamine (0.10 ml, 0.97 mmol) and 3-ethynyl-phenylamine (0.126 mg, 0.0011 mmol) in dimethylformamide (1 ml) and toluene (10 ml) and the mixture stirred at reflux for 2 h. Water (20 ml) was added and the organic phase was extracted and washed with water (20 ml) then dried over MgSO₄. Concentration *in vacuo* produced a mixture of lidocaine and tagged lidocaine (0.1 mol %) as a white crystalline solid (0.179 mg, 0.76 mmol, 79 %).

An un-tagged lidocaine product was made by the following method: Diethylamine (0.39 ml, 3.74 mmol) was added to a solution of 2-chloro-N-(2,6-dimethyl-phenyl)-acetamide (0.25 g, 1.25 mmol) in toluene (10 ml) and the mixture stirred at reflux for 3 h. After cooling to room temperature the reaction was quenched with aq. HCI (10%, 10 ml). The aqueous phase was collected and basified with aq. NaOH until strongly basic. Ethyl acetate (20 ml) was added and the organic layer removed, dried over MgSO₄ and concentrated in vacuo. Recrystallisation from n-pentane afforded lidocaine (0.11 g, 0.47 mmol, 38 %) as white, needle-like, crystals. The product was confirmed by GC-MS and NMR.

Both 0.1 mol% marked and unmarked Lidocaine (30 mg) were separately taken up in water (3 ml) and acetone (0.1 ml) and shaken to form a stock solution. Gold colloid (500 µL, 60.7 nm in citrate buffer, 0.01% w/w supplied by Cabot) and deionised water (300 µL) were added to 100 µL of the above stock solution and the shaken. NaCl (100 µL, 5% aq.) was added, the mixture shaken and the SERS response of the resulting solution was then collected using an Ocean Optics 785 nm laser (QE65000, 1000 ms integration, 5 iterations). The spectra are shown in Fig 2. Distinct SERS bands can be seen in the tagged sample at 1157, 1574 and 1590 cm-1.

## Claims

1. A process for marking a composition comprising a chemical product C and identifying and distinguishing the marked composition from similar compositions, the process comprising the steps of:
(i) forming a reaction mixture comprising chemical intermediate A, chemical intermediate B and marker chemical M;
(ii) providing the conditions which enable chemical intermediate A and chemical intermediate B to react together to form chemical product C, and for marker chemical M to react with one or both of chemical intermediate A to form a marker product AM and chemical product C to form a marker product CM;
(iii) after said reactions have taken place, separating a mixture of chemical product C with marker product AM and/or a mixture of chemical product C with marker product CM from the reaction mixture to obtain said marked composition, wherein marker product AM and marker product CM are both distinguishable from chemical product C by analysis of said composition;
(iv) **characterized in that** the process further comprises determining the absolute and relative amounts of said marker product CM or the marker product AM and the product C in said marked composition and using the absolute or relative amounts to identify and distinguish said marked composition.

2. A process as claimed in claim 1, wherein chemical intermediate B incorporates a functional group capable of reacting with chemical intermediate A and marker chemical M incorporates the same type of functional group.

3. A process as claimed in claim 1, wherein chemical intermediate B incorporates a functional group capable of reacting with chemical intermediate A and marker chemical M incorporates a different type of functional group capable of reacting with chemical intermediate A or chemical product C.

4. A process as claimed in any one of the preceding claims, wherein the mixture of chemical product C with marker product AM or CM separated from the reaction mixture in step (iii) of claim 1, is a homogeneous mixture.

5. A process as claimed in any one of the preceding claims, wherein said analysis comprises at least one analytical method selected from spectroscopy and chromatography.

6. A process as claimed in claim 5, wherein said analytical method is selected from the group consisting
of spectrophotometric absorption in the ultraviolet, visible, near infrared or mid-infrared regions; fluorescent emission spectroscopy; nuclear magnetic resonance spectroscopy, electron spin resonance spectroscopy, Raman spectroscopy, resonance Raman spectroscopy, surface-enhanced Raman spectroscopy (SERS); surface-enhanced resonance Raman spectroscopy (SERRS), tip enhanced Raman spectroscopy, spatially off-set Raman spectroscopy, gas chromatography, liquid chromatography or HPLC.

7. A process as claimed in any one of the preceding claims, wherein chemical product C is a herbicide, a pesticide, an active pharmaceutical ingredient (API), a food ingredient, a dye, a pigment, an aroma compound, a fuel additive, a polymer or a plant product.

8. A process as claimed in any one of the preceding claims, wherein chemical product C comprises an ingredient in a composition.

9. A process as claimed in any one of the preceding claims, wherein said mixture contains from 1 ppb by weight to 1% by weight of marker product AM or marker product CM based on the total weight of chemical product C and said marker product.

10. A process as claimed in any one of the preceding claims, wherein at least two marker chemicals M1, M2 ...Mn are present in said reaction mixture, where n represents the number of marker chemicals M present in the mixture, each said marker chemical M forming a different marker product and each said different marker product being distinguishable from chemical product C and from each other marker product present in the mixture by analysis.

11. A process as claimed in claim 10, wherein the relative proportions of marker chemicals M1 - Mn are
calculated to provide an identifiable signal ratio of each marker product when said mixture is analysed.

## Patentansprüche

1. Verfahren zum Markieren einer Zusammensetzung, umfassend ein chemisches Produkt C und Identifizieren und Unterscheiden der markierten Zusammensetzung von ähnlichen Zusammensetzungen, wobei das Verfahren die folgenden Schritte umfasst:
(i) Bilden eines Reaktionsgemisches, ein chemisches Zwischenprodukt A, ein chemisches Zwischenprodukt B und eine Markierchemikalie M umfassend;
(ii) Bereitstellen der Bedingungen, die es ermöglichen, dass das chemische Zwischenprodukt A und das chemische Zwischenprodukt B miteinander reagieren, um ein chemisches Produkt C zu bilden, und für die Markierchemikalie M, um mit einem oder beiden des chemischen Zwischenprodukts A zu reagieren, um ein Markierprodukt AM zu bilden, und des chemischen Produkts C zu reagieren, um ein Markierprodukt CM zu bilden;
(iii) nachdem die Reaktionen stattgefunden haben, Abscheiden eines Gemisches eines chemischen Produkts C mit dem Markierprodukt AM und/oder eines Gemisches eines chemischen Produkts C mit dem Markierprodukt CM von dem Reaktionsgemisch, um die markierte Zusammensetzung zu erhalten,
wobei das Markierprodukt AM und Markierprodukt CM beide von dem chemischen Produkt C durch Analysieren der Zusammensetzung unterscheidbar sind;
(iv) **dadurch gekennzeichnet, dass** das Verfahren weiter das Bestimmen der absoluten und relativen Mengen des Markierprodukts CM oder des Markierprodukts AM und des Produkts C in der markierten Zusammensetzung umfasst und das Verwenden der absoluten und relativen Mengen zum Identifizieren und Unterscheiden der markierten Zusammensetzung.

2. Verfahren nach Anspruch 1, wobei das chemische Zwischenprodukt B eine funktionelle Gruppe beinhaltet, die imstande ist, mit dem chemischen Zwischenprodukt A zu reagieren, und die Markierchemikalie M dieselbe Art von funktioneller Gruppe beinhaltet.

3. Verfahren nach Anspruch 1, wobei das chemische Zwischenprodukt B eine funktionelle Gruppe beinhaltet, die imstande ist, mit dem chemischen Zwischenprodukt A zu reagieren, und die Markierchemikalie M eine andere Art einer funktionellen Gruppe beinhaltet, die imstande ist, mit dem chemischen Zwischenprodukt A oder dem chemischen Produkt C zu reagieren.

4. Verfahren nach einem der vorstehenden Ansprüche, wobei das Gemisch eines chemischen Produkts C mit dem Markierprodukt AM oder CM, das in Schritt (iii) nach Anspruch 1 von dem Reaktionsgemisch abgeschieden worden ist, ein homogenes Gemisch ist.

5. Verfahren nach einem der vorstehenden Ansprüche, wobei die Analyse mindestens eine analytische Methode umfasst, die aus der Spektroskopie und Chromatographie ausgewählt wird.

6. Verfahren nach Anspruch 5,
wobei die analytische Methode aus der Gruppe ausgewählt ist, bestehend aus spektrophotometrischer Absorption in den ultravioletten, sichtbaren, nahinfraroten oder mittel-infraroten Bereichen; fluoreszierender Emissionsspektroskopie; Kernspinresonanzspektroskopie, Elektronenspin-Resonanzspektroskopie, Raman-Spektroskopie, Raman Resonanzspektroskopie, Oberflächen-verstärkte Raman-Spektroskopie (SERS); Oberflächen-verstärkte Raman Resonanzspektroskopie (SERRS), spitzenverstärkte Raman-Spektroskopie, räumlich versetzte Raman-Spektroskopie, Gaschromatographie, Flüssigchromatographie oder HPLC.

7. Verfahren nach einem der vorstehenden Ansprüche, wobei das chemische Produkt C ein Herbizid, ein Pestizid, ein pharmazeutischer Wirkstoff (API), eine Lebensmittelzutat, ein Farbstoff, ein Pigment, eine Aromaverbindung, ein Treibstoffadditiv, ein Polymer oder ein Pflanzenprodukt ist.

8. Verfahren nach einem der vorstehenden Ansprüche, wobei das chemische Produkt C eine Zutat in einer Zusammensetzung umfasst.

9. Verfahren nach einem der vorstehenden Ansprüche, wobei das Gemisch von 1 Gewicht-ppb bis 1 Gew.-% eines Markierprodukts AM oder Markierprodukts CM basierend auf dem Gesamtgewicht des chemischen Produkts C und Markierprodukts enthält.

10. Verfahren nach einem der vorstehenden Ansprüche, wobei mindestens zwei Markierchemikalien M1, M2 ...Mn in dem Reaktionsgemisch vorhanden sind, wobei n die Anzahl an Markierchemikalien M darstellt, die in dem Gemisch vorhanden sind, wobei jede Markierchemikalie M ein anderes Markierprodukt bildet und jedes unterschiedliche Markierprodukt durch Analyse vom chemischen Produkt C und von jedem anderen Markierprodukt, das in dem Gemisch vorhanden ist, unterscheidbar ist.

11. Verfahren nach Anspruch 10,
wobei die relativen Proportionen von Markierchemikalien M1 - Mn berechnet werden, um ein identifizierbares Signalverhältnis jedes Markierprodukts bereitzustellen, wenn das Gemisch analysiert wird.

## Revendications

1. Procédé pour marquer une composition comprenant un produit chimique C et identifier et distinguer la composition marquée de compositions similaires, le procédé comprenant les étapes :
(i) former un mélange réactionnel comprenant un intermédiaire chimique A, un intermédiaire chimique B et un marqueur chimique M ;
(ii) fournir les conditions qui permettent à l'intermédiaire chimique A et à l'intermédiaire chimique B de réagir ensemble pour former le produit chimique C, et au marqueur chimique M de réagir avec l'un ou l'autre ou les deux parmi l'intermédiaire A pour former un produit marqueur AM et le produit chimique C pour former un produit marqueur CM ;
(iii) une fois que lesdites réactions ont eu lieu, séparer un mélange du produit chimique C avec le produit marqueur AM et/ou un mélange du produit chimique C avec le produit marqueur CM à partir du mélange réactionnel, pour obtenir ladite composition marquée,
le produit marqueur AM et le produit marqueur CM pouvant tous deux être distingués du produit chimique C par analyse de ladite composition ;
(iv) **caractérisé en ce que** le procédé comprend en outre la détermination des quantités absolues et relatives dudit produit marqueur CM ou du produit marqueur AM et du produit C dans ladite composition marquée et l'utilisation des quantités absolues ou relatives pour identifier et distinguer ladite composition marquée.

2. Procédé selon la revendication 1, dans lequel l'intermédiaire chimique B renferme un groupe fonctionnel capable de réagir avec l'intermédiaire chimique A et le marqueur chimique M renferme le même type de groupe fonctionnel.

3. Procédé selon la revendication 1, dans lequel l'intermédiaire chimique B renferme un groupe fonctionnel capable de réagir avec l'intermédiaire chimique A et le marqueur chimique M renferme un type différent de groupe fonctionnel capable de réagir avec l'intermédiaire chimique A ou le produit chimique C.

4. Procédé selon l'une quelconque des revendications précédentes, dans lequel le mélange du produit chimique C avec le produit marqueur AM ou CM séparé du mélange réactionnel à l'étape (iii) de la revendication 1, est un mélange homogène.

5. Procédé selon l'une quelconque des revendications précédentes, dans lequel ladite analyse comprend au moins une méthode analytique choisie parmi la spectroscopie et la chromatographie.

6. Procédé selon la revendication 5, dans lequel ladite méthode analytique est choisie dans le groupe constitué par l'absorption spectrophotométrique dans les régions de l'ultraviolet, du visible, du proche infrarouge ou de l'infrarouge moyen ; la spectroscopie d'émission de fluorescence ; la spectroscopie par résonance magnétique nucléaire, la spectroscopie par résonance de spin électronique, la spectroscopie Raman, la spectroscopie Raman de résonance, la spectroscopie Raman exaltée de surface (SERS) ; la spectroscopie Raman de résonance exaltée de surface (SERRS), la spectroscopie Raman exaltée par effet de pointe, la spectroscopie Raman à décalage spatial, la chromatographie en phase gazeuse, la chromatographie en phase liquide ou la HPLC.

7. Procédé selon l'une quelconque des revendications précédentes, dans lequel le produit chimique C est un herbicide, un pesticide, un principe actif pharmaceutique (API), un ingrédient alimentaire, un colorant, un pigment, un composé d'arôme, un additif pour carburant, un polymère ou un produit végétal.

8. Procédé selon l'une quelconque des revendications précédentes, dans lequel le produit chimique C comprend un ingrédient dans une composition.

9. Procédé selon l'une quelconque des revendications précédentes, dans lequel ledit mélange contient de 1 ppb en poids à 1 % en poids de produit marqueur AM ou de produit marqueur CM rapporté au poids total du produit chimique C et dudit produit marqueur.

10. Procédé selon l'une quelconque des revendications précédentes, dans lequel au moins deux marqueurs chimiques M1, M2... Mn sont présents dans ledit mélange réactionnel, où n représente le nombre de marqueurs chimiques M présents dans le mélange, chacun desdits marqueurs chimiques M formant un produit marqueur différent et chacun desdits produits marqueurs différents pouvant être distingué du produit chimique C et de chacun des autres produits marqueurs présents dans le mélange par analyse.

11. Procédé selon la revendication 10, dans lequel les proportions relatives de marqueurs chimiques M1 - Mn sont calculées pour fournir un rapport de signaux identifiable propre à chaque produit marqueur lorsque ledit mélange est analysé.
